Europäisches Patentamt

European Patent Office

Office européen des brevets

(19)

(11) Veröffentlichungsnummer: **0 024 555**
**B1**

(12) **EUROPÄISCHE PATENTSCHRIFT**

(45) Veröffentlichungstag der Patentschrift:
15.09.82

(51) Int. Cl.³: **C 07 C 119/048**, C 07 C 118/00,
C 08 G 18/76

(21) Anmeldenummer: 80104406.6

(22) Anmeldetag: 26.07.80

(54) **Verfahren zur Herstellung von Diisocanatotoluolgemischen mit einem erhöhten Gehalt an 2,6-Diisocyanatotoluol sowie Herstellung von Polyurethanelastomeren unter Verwendung dieser Gemische.**

(30) Priorität: 08.08.79 DE 2932095

(43) Veröffentlichungstag der Anmeldung:
11.03.81 Patentblatt 81/10

(45) Bekanntmachung des Hinweises auf die Patenterteilung:
15.09.82 Patentblatt 82/37

(84) Benannte Vertragsstaaten:
BE DE FR GB IT

(56) Entgegenhaltungen:
DE-A-2 063 731
DE-B-1 693 220
US-A-3 316 285

(73) Patentinhaber: BAYER AG, Zentralbereich Patente,
Marken und Lizenzen, D-5090 Leverkusen 1, Bayerwerk
(DE)

(72) Erfinder: Mazanek, Jan, Dr., Görlitzer Strasse 9,
D-8000 München 50 (DE)
Erfinder: Müller, Hanns Peter, Dr., St.
Pankratius-Strasse 2, D-5068 Odenthal (DE)

BUNDESDRUCKEREI BERLIN

Verfahren zur Herstellung von Diisocyanatotoluolgemischen mit einem erhöhten Gehalt
an 2,6-Diisocyanatotoluol, sowie Herstellung von
Polyurethanelastomeren unter Verwendung dieser Gemische

Die vorliegende Erfindung betrifft ein neues Verfahren zur Herstellung von Gemischen aus 2,4- und 2,6-Diisocyanatotoluol, die durch einen besonders hohen Gehalt an 2,6-Diisocyanatotoluol gekennzeichnet sind, sowie die Verwendung dieser Gemische als Aufbaukomponente zur Herstellung von Polyurethanelastomeren.

Diisocyanatotoluole oder auch Toluylendiisocyanate (abgekürzt: TDI) sind technisch und wirtschaftlich äußerst wichtige Ausgangsmaterialien zur großtechnischen Herstellung von Polyurethankunststoffen. Sie werden großtechnisch durch Phosgenierung der entsprechenden Diaminotoluole bzw. Toluylendiamine (abgekürzt: TDA) gewonnen. Bislang spielten großtechnisch insbesondere »TDI 100«, »TDI 80« und »TDI 65« eine Rolle, d. h. reines 2,4-Diisocyanatotoluol bzw. dessen Abmischungen mit 20 bzw. 35 Gew.-%, bezogen auf Gesamtgemisch, an 2,6-Diisocyanatotoluol.

Auch TDI mit einem über 35 Gew.-% liegenden Gehalt an 2,6-Diisocyanattoluol ist bereits bekanntgeworden. So beschreibt beispielsweise US 3 022 328 die partielle Umsetzung von TDI 65 mit tert. Alkoholen und destillative Isolierung von, an 2,6-Diisocyanatotoluol angereichertem, Toluylendiisocyanat-Isomerengemisch aus dem erhaltenen Umsetzungsprodukt. Gemäß US-PS 3 554 872 erfolgt eine analoge Anreicherung des 2,6-Isomeren durch partielle Umsetzung von TDI mit Polyester- oder Polyetherpolyolen und anschließende destillative Aufarbeitung des Umsetzungsprodukts.

Wie nun gefunden wurde, gelingt die Herstellung von Diisocyanatotoluol-Isomerengemischen mit einem Gehalt von 36 bis 90, vorzugsweise 45 bis 85 Gew.-% an 2,6-Diisocyanatotoluol aus Diisocyanatotoluol-Isomerengemischen mit einem Gehalt an 2,6-Diisocyanatotoluol von maximal 35 Gew.-% auf besonders einfache Weise durch partielle Trimerisierung der Isocyanatgruppen des Ausgangsgemisches und anschließende Isolierung des nicht umgesetzten, an 2,6-Diisocyanatotoluol angereichertem Diisocyanat. Außerdem wurde gefunden, daß die so oder nach beliebigen Verfahren des Standes der Technik erhältlichen Diisocyanatotoluol-Isomerengemische mit einem Gehalt an 2,6-Diisocyanatotoluol von insbesondere 45 bis 85 Gew.-% mit den üblichen höhermolekularen Polyhydroxylverbindungen der Polyurethanchemie und Kettenverlängerungsmitteln auch dann zu hochwertigen Polyurethanelastomeren verarbeitet werden können, wenn als Kettenverlängerungsmittel anstelle der üblichen Diamine einfache Glykole eingesetzt werden. Bislang war die Herstellung hochwertiger Polyurethanelastomerer aus TDI, höhermolekularem Polyol und niedermolekularen Glykolen als Kettenverlängerungsmittel nicht möglich; vielmehr mußte man bislang zur Herstellung derartiger Elastomerer entweder teure und oft physiologisch bedenkliche Diamin-Kettenverlängerungsmittel oder spezielle Diisocyanate wie z. B. 1,5-Diisocyanato-naphthalin einsetzen, um den Anforderungen der Praxis an das mechanische Werteniveau von Polyurethanelastomeren gerecht zu werden.

Gegenstand der vorliegenden Erfindung ist somit ein Verfahren zur Herstellung von im wesentlichen aus 2,4-Diisocyanatotoluol und 2,6-Diisocyanatotoluol bestehenden Diisocyanatgemischen mit einem Gehalt an 2,6-Diisocyanatotoluol, bezogen auf Gesamtgemisch von 36 bis 90, vorzugsweise 45 bis 85 Gew.-%, welches dadurch gekennzeichnet ist, daß man einen Teil der Isocyanatgruppen eines im wesentlichen aus 2,4-Diisocyanatotoluol und 2,6-Diisocyanatotoluol bestehenden Diisocyanatgemischs mit einem Gehalt von maximal 35 Gew.-%, bezogen auf Gesamtgemisch, an 2,6-Diisocyanatotoluol einer Trimerisierungsreaktion unterzieht und aus dem so erhaltenen Reaktionsgemisch ein Diisocyanatgemisch mit einem erhöhten Gehalt an 2,6-Diisocyanatotoluol isoliert.

Gegenstand der vorliegenden Erfindung ist schließlich auch ein Verfahren zur Herstellung von Polyurethanelastomeren durch Umsetzung von organischen Polyisocyanaten mit höhermolekularen Polyhydroxylverbindungen und niedermolekularen Kettenverlängerungsmitteln nach dem Ein- oder Zweistufen-Prinzip, welches dadurch gekennzeichnet ist, daß man

a) als Polyisocyanatkomponente im wesentlichen aus 2,4-Diisocyanatotoluol und 2,6-Diisocyanatotoluol bestehende Diisocyanatgemische mit einem Gehalt an 2,6-Diisocyanatotoluol, bezogen auf Gesamtgemisch, von 36 bis 90, vorzugsweise 45 bis 85 Gew.-% und

b) als Kettenverlängerungsmittel gegebenenfalls Etherbrücken aufweisende aliphatische Glykole des Molekulargewichtsbereich 62 bis 200 verwendet.

Ausgangsmaterialien für das erfindungsgemäße Verfahren zur Herstellung der an 2,6-Diisocyanatotoluol angereicherten Isomerengemische sind im wesentlichen aus 2,4-Diisocyanatotoluol und 2,6-Diisocyanatotoluol bestehende Diisocyanatgemische mit einem Gehalt an 2,6-Diisocyanatotoluol von maximal 35 Gew.-%, bezogen auf Gesamtgemisch. Bevorzugte Ausgangsmaterialien sind derartige Gemische mit einem Gehalt an 2,6-Diisocyanatotoluol, bezogen auf Gesamtgemisch von 20 bzw. 35 Gew.-%. Bei diesem Ausgangsmaterialien für das erfindungsgemäße Verfahren handelt es

2

sich vorzugsweise um die aus der Polyurethanchemie seit langen Jahren bekannten Diisocyanatotoluol-Gemische, die im allgemeinen zu mindestens 99 Gew.-%, bezogen auf Gesamtgemisch, aus den genannten Isomeren bestehen.

Zur erfindungsgemäßen Trimerisierung eines Teils der Isocyanatgruppen des als Ausgangsmaterial dienenden Diisocyanats bedient man sich der an sich bekannten Methoden zur Trimerisierung von organischen Diisocyanaten wie sie im Prinzip beispielsweise in »Polyurethanes«, I., Interscience Publishers (1962), Seiten 94—97 beschrieben werden. Auch hier können beliebige, durch Katalysatorengifte oder thermisch desaktivierbare Katalysatoren verwendet werden. Gut geeignet ist beispielsweise auch hier die Verwendung von tert.-Phosphinen wie z. B. Tributylphosphin als Katalysator und von Schwefel als Katalysatorgift, wie dies in GB-PS 1 244 416 oder DE-AS 1 954 093 beschrieben ist. Die Reaktion wird bei erhöhten Temperaturen, beispielsweise bei 30 bis 150°C, vorzugsweise bei 50 bis 120°C durchgeführt. Die Mitverwendung eines Hilfslösungsmittels wie beispielsweise Ethylacetat oder Butylacetat kann zweckmäßig sein. Wie in Beispiel 3 der DE-AS 1 954 093 beschrieben, können nach beendigter Umsetzung geringe Mengen an nicht umgesetztem Schwefel durch Filtration entfernt werden, worauf sich eine destillative Aufarbeitung des Reaktionsgemisches anschließt. Ebenfalls gut geeignet sind beispielsweise die Katalysatoren der DE-OS 2 551 634 (US 4 115 373). So ist beispielsweise die Mannichbase, die durch Kondensation von 2 Mol Phenol mit 4 Mol Methylamin und ca. 5,5 Mol Formaldehyd gemäß Beispiel 1 der DE-OS 2 551 634 zugänglich ist, auch für das erfindungsgemäße Verfahren besonders gut geeignet. Die Trimerisierungsreaktion wird im Temperaturbereich von ca. 30 bis 150°C, vorzugsweise 30 bis 100°C durchgeführt. Die Abstoppung der Reaktion erfolgt entweder durch Zugabe eines Katalysatorengifts wie z. B. Toluolsulfonsäuremethylester oder durch Erhitzen des Reaktionsgemischs auf ca. 110—150°C, vorzugsweise 110—120°C unter thermischer Zersetzung des Katalysators. Auch hier kann die Mitverwendung eines Hilfslösungsmittels wie z. B. von Ethylacetat oder Butylacetat zweckmäßig sein. Die Trimerisierungsreaktion kann jedoch auch in Substanz durchgeführt werden. Die Trimerisierungskatalysatoren werden im allgemeinen in einer Menge von 0,01 bis 3 Gew.-%, bezogen auf Ausgangsisocyanat, eingesetzt. Oft erweist sich ein Zusatz einer geringen Menge einer sauren Verbindung wie z. B. Benzoylchlorid zum Ausgangsisocyanat als sinnvoll, um Spuren basischer Verbindungen, die Nebenreaktionen katalysieren könnten, zu neutralisieren.

Das erfindungsgemäße Verfahren wird im allgemeinen bei einer Modifizierung von 5 bis 45%, vorzugsweise 10 bis 40% der im Ausgangsgemisch vorliegenden Isocyanatgruppen abgebrochen. Das unveränderte Diisocyanat wird dann aus dem Reaktionsgemisch beispielsweise durch Extraktion oder vorzugsweise durch Destillation isoliert. Die Frage, wie weit die erfindungswesentliche Ummodifizierungsreaktion geführt wird, hängt selbstverständlich vom Gehalt des Ausgangsgemisches an 2,6-Diisocyanatotoluol und vom angestrebten Gehalt des Verfahrensprodukt an diesem Isomeren ab. Die erfindungsgemäßen Verfahrensprodukte bestehen im wesentlichen aus 36 bis 90, vorzugsweise 45 bis 85 Gew.-% 2,6-Diisocyanatotoluol und 10 bis 64, vorzugsweise 15 bis 55 Gew.-% an 2,4-Diisocyanatotoluol.

Für das erfindungsgemäße Verfahren zur Herstellung von Polyurethanelastomeren werden die so oder nach beliebigen anderen Verfahren erhaltenen Diisocyanatotoluol-Isomerengemische der genannten Zusammensetzung mit höhermolekularen Polyhydroxylverbindungen und ausgewählten Kettenverlängerungsmitteln zur Reaktion gebracht.

Geeignete höhermolekulare Hydroxylverbindungen sind 2 bis 4, vorzugsweise 2 oder 3, besonders bevorzugt 2 Hydroxylgruppen aufweisende Polyester, Polyether, Polythioether, Polyacetale, Polycarbonate und Polyesteramide mit niedrigem Schmelzpunkt, wie sie für die Herstellung von Polyurethanen an für sich bekannt sind. Diese Polyhydroxylverbindungen haben im allgemeinen ein Molekulargewicht von 400 bis 10 000, vorzugsweise von 800 bis 6000 und einem Schmelzpunkt unterhalb 60°C, vorzugsweise unterhalb 40°C, besonders bevorzugt unterhalb 30°C. Die entsprechenden Hydroxylgruppen aufweisenden Polyester, Polyether oder Polycarbonate sind bevorzugt.

Die in Frage kommenden Hydroxylgruppen aufweisenden Polyester sind z. B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z. B. durch Halogenatome, substituiert und/oder ungesättigt sein.

Als Beispiele hierfür seien genannt: Adipinsäure, Phthalsäure, Isophthalsäure.

Als mehrwertige Alkohole kommen z. B. Ethylenglykol, Propylenglykol-(1,2) und -(1,3), Butylenglykol-(1,4) und -(2,3), Hexandiol-(1,6), Octandiol-(1,8), Neopentylglykol, Cyclohexandimethanol(1,4-Bis-(hydroxymethyl)-cyclohexan, 2-Methyl-1,3-propandiol, Glycerin oder Trimethylolpropan in Frage.

Auch die Hydroxylgruppen aufweisenden Polyether sind solche der an sich bekannten Art und werden z. B. durch Polymerisation von Epoxiden wie Ethylenoxid, Propylenoxid, Butylenoxid, Tetrahydrofuran, Styroloxid oder Epichlorhydrin mit sich selbst, z. B. in Gegenwart von $BF_3$, oder durch

3

Anlagerung dieser Epoxide, gegebenenfalls im Gemisch oder nacheinander, an Startkomponenten mit reaktionsfähigen Wasserstoffatomen wie Wasser, Alkohole, Ammoniak oder Amine, z. B. Äthylenglykol, Propylenglykol-(1,3) oder -(1,2), Trimethylolpropan, 4,4'-Dihydroxy-diphenylpropan, Anilin, Äthanolamin oder Äthylendiamin hergestellt. Vielfach sind solche Polyäther bevorzugt, die überwiegend (bis zu 90 Gew.-%, bezogen auf alle vorhandenen OH-Gruppen im Polyäther) primäre OH-Gruppen aufweisen. Auch durch Vinylpolymerisate modifizierte Polyäther, wie sie z. B. durch Polymerisation von Styrol und Acrylnitril in Gegenwart von Polyäthern entstehen (amerikanische Patentschriften 3 383 351, 3 304 273, 3 523 093, 3 110 695, deutsche Patentschrift 1 152 536), sind geeignet.

Vertreter dieser erfindungsgemäß zu verwendenden Verbindungen sind z. B. in High Polymers, Vol. XVI, »Polyurethanes, Chemistry and Technology«, verfaßt von Saunders-Frisch, Interscience Publishers, New York, London, Band I, 1962, Seiten 32—42 und Seiten 44—54 und Band II, 1964, Seiten 5—6 und 198—199, sowie im Kunststoff-Handbuch, Band VII, Vieweg-Höchtlen, Carl-Hanser-Verlag, München, 1966, z. B. auf den Seiten 45—71, beschrieben.

Selbstverständlich können Mischungen der obengenannten Verbindungen, z. B. Mischungen von Polyäthern und Polyestern, eingesetzt werden.

Erfindungsgemäß können jedoch auch Polyhydroxylverbindungen eingesetzt werden, in welchen hochmolekulare Polyaddukte bzw. Polykondensate in feindisperser oder gelöster Form enthalten sind. Derartige modifizierte Polyhydroxylverbindungen werden erhalten, wenn man Polyadditionsreaktionen (z. B. Umsetzungen zwischen Polyisocyanaten und aminofunktionellen Verbindungen) bzw. Polykondensationsreaktionen (z. B. zwischen Formaldehyd und Phenolen und/oder Aminen) direkt in situ in den obengenannten, Hydroxylgruppen aufweisenden Verbindungen ablaufen läßt. Derartige Verfahren sind beispielsweise in den deutschen Auslegeschriften 1 168 075 und 1 260 142, sowie den deutschen Offenlegungsschriften 2 324 134, 2 423 984, 2 512 385, 2 513 815, 2 550 796, 2 550 797, 2 550 833 und 2 550 862 beschrieben. Es ist aber auch möglich, gemäß US-Patent 3 869 413 bzw. Deutscher Offenlegungsschrift 2 550 860 eine fertige wäßrige Polymerdispersion mit einer Polyhydroxylverbindung zu vermischen und anschließend aus dem Gemisch das Wasser zu entfernen.

Als Hydroxylgruppen aufweisende Polycarbonate kommen solche der an sich bekannten Art in Betracht, die z. B. durch Umsetzung von Diolen wie Propandiol-(1,3), Butandiol-(1,4) und/oder Hexandiol-(1,6), Diethylenglykol, Triethylenglykol oder Tetraethylenglykol mit Diarylcarbonaten z. B. Diphenylcarbonat oder Phosgen hergestellt werden können.

Als Kettenverlängerungsmittel kommen beim erfindungsgemäßen Verfahren zur Herstellung von Polyurethanelastomeren beliebige, gegebenenfalls durch Etherbrücken unterbrochene aliphatische Glykole des Molekulargewichtsbereichs 62 bis 200 in Betracht. Als Beispiele für derartige Verbindungen seien genannt: Ethylenglykol, Propylenglykol-(1,2) und -(1,3), Butylenglykol-(1,4) und -(2,3), Pentandiol-(1,5), Hexandiol-(1,6), Neopentylglykol, Diethylenglykol, Triethylenglykol, Dipropylenglykol, Tripropylenglykol, Dibutylenglykol oder Tributylenglykol. Gemische der genannten Kettenverlängerungsmittel können selbstverständlich auch eingesetzt werden.

Bei der Durchführung des erfindungsgemäßen Verfahrens zur Herstellung von Polyurethanelastomeren kann nach dem Ein- oder dem Zweistufen-Prinzip verfahren werden. Bei der Herstellung der Polyurethanelastomeren nach dem Einstufen-Prinzip werden die erfindungswesentlichen Diisocyanate mit einem Gemisch aus höhermolekulare Polyhydroxylverbindung und Kettenverlängerungsmittel zur Reaktion gebracht. Die Mengenverhältnisse der Reaktionspartner werden dabei so gewählt, daß für jedes Mol an höhermolekularer Polyhydroxylverbindung 0,5 bis 7, vorzugsweise 1 bis 3 Mol Kettenverlängerungsmittel vorliegen und ein NCO/OH-Äquivalentverhältnis, bezogen auf alle Isocyanat- und alle Hydroxylgruppen von 0,8 bis 2, vorzugsweise 0,9 bis 1,3 gewährleistet ist. Die Umsetzung nach dem Einstufen-Prinzip erfolgt im allgemeinen lösungsmittelfrei bei 80 bis 150, vorzugsweise 90 bis 120°C. Bei der Durchführung des erfindungsgemäßen Verfahrens zur Herstellung von Polyurethanelastomeren nach dem Zweistufen-Prinzip wird zunächst aus dem Diisocyanat und der höhermolekularen Polyhydroxylverbindung bei 70 bis 140°C, vorzugsweise 90 bis 120°C ein NCO-Präpolymer hergestellt, wobei die Reaktionspartner in einer Menge zum Einsatz gelangen, die einem NCO/OH-Äquivalentverhältnis von 1,5 bis 6, vorzugsweise 2 bis 4 entsprechen. Die Kettenverlängerungsreaktion erfolgt anschließend durch Zugabe des Kettenverlängerungsmittels zum so erhaltenen NCO-Präpolymer, wobei die Menge des Kettenverlängerungsmittels so bemessen wird, daß, bezogen auf NCO-Gruppen des NCO-Präpolymeren und Hydroxylgruppen des Kettenverlängerungsmittels, ein NCO/OH-Äquivalentverhältnis von 0,8 bis 2, vorzugsweise 0,9 bis 1,3 gewährleistet ist. die Kettenverlängerungsreaktion wird im allgemeinen im Temperaturbereich von 80 bis 150, vorzugsweise 90 bis 120°C durchgeführt. Bei der Herstellung der Polyurethanelastomeren werden oft Katalysatoren mitverwendet. Als mitzuverwendende Katalysatoren kommen solche der an sich bekannten Art in Frage, z. B. tertiäre Amine, wie Triethylamin, 1,4-Diaza-bicyclo-(2,2,2)-octan, Aminoethyl-piperazin oder N,N-Dimethylbenzylamin.

Erfindungsgemäß können auch organische Metallverbindungen, insbesondere organische Zinnverbindungen, als Katalysatoren, verwendet werden.

Als organische Zinnverbindungen kommen vorzugsweise Zinn(II)-salze von Carbonsäuren wie Zinn(II)-acetat, Zinn(II)-octoat, Zinn(II)-ethylhexoat und Zinn(II)-laurat und die Zinn(IV)-Verbindungen, z. B. Dibutylzinnoxid, Dibutylzinndichlorid, Dibutylzinndiacetat, Dibutylzinndilaurat, Dibutylzinnmaleat oder Dioctylzinndiacetat in Betracht. Selbstverständlich können alle obengenannten Katalysatoren als Gemische eingesetzt werden.

Weitere Vertreter von erfindungsgemäß zu verwendenden Katalysatoren sowie Einzelheiten über die Wirkungsweise der Katalysatoren sind im Kunststoff-Handbuch, Band VII, herausgegeben von Vieweg und Höchtlen, Carl-Hanser-Verlag, München 1966, z. B. auf den Seiten 96 bis 102 beschrieben.

Die Katalysatoren werden in der Regel in einer Menge zwischen etwa 0,001 und 10 Gew.-%, bezogen auf die Menge an Verbindungen mit mindestens zwei gegenüber Isocyanaten reaktionsfähigen Wasserstoffatomen eingesetzt.

Erfindungsgemäß können ferner auch Reaktionsverzögerer, z. B. sauer reagierende Stoffe wie Salzsäure oder organische Säurehalogenide, Flammschutzmittel der an sich bekannten Art, z. B. Tris-chlorethylphosphat, Trikresylphosphat oder Ammoniumphosphat und -polyphosphat, ferner Stabilisatoren gegen Alterungs- und Witterungseinflüssen, Weichmacher und fungistatisch und bakteriostatisch wirkende Substanzen, Füllstoffe wie Bariumsulfat, Kieselgur, Ruß oder Schlämmkreide mitverwendet werden.

Beispiele von gegebenenfalls erfindungsgemäß mitzuverwendeten Hilfs- und Zusatzstoffen sowie Einzelheiten über Verwendungs- und Wirkungsweise dieser Zusatzmittel sind im Kunststoff-Handbuch, Band VII, herausgegeben von Vieweg und Höchtlen, Carl-Hanser-Verlag, München 1966, z. B. auf den Seiten 103 bis 113 beschrieben.

Die nach dem erfindungsgemäßen Verfahren erhältlichen Polyurethanelastomeren zeichnen sich trotz der Verwendung von Diisocyanatotoluol als Polyisocyanatkomponente und gleichzeitig von einfachen Glykolen als Kettenverlängerungsmittel durch ausgezeichnete mechanischen Eigenschaften aus.

Die nachfolgenden Beispiele dienen zur weiteren Erläuterung der Erfindung, ohne jedoch diese zu beschränken.

In den Herstellungsbeispielen für die Diisocyanatgemische mit angereichertem Gehalt an 2,6-Isomeren werden jeweils Ausgangsgemische mit einem Gehalt von 2,4- und 2,6-Diisocyanatotoluol von mindestens 99 Gew.-% eingesetzt. Alle Prozentangaben beziehen sich auf Gew.-%.

### Beispiel 1

730 g Toluylendiisocyanat (80% 2,4, 20% 2,6-Isomeres) und 0,7 g Benzoylchlorid wurden unter Stickstoffatmosphäre auf 80°C erhitzt. 2,4 g der Mannichbase gemäß Beispiel 1 der DE-OS 2 551 634 (55%iges Gemisch) wurden zugegeben, die Reaktionstemperatur stieg innerhalb von etwa 10 Minuten auf 105°C. Anschließend wurde 50 Minuten bei 110°C und dann 5 Minuten bei 120°C weiter unter Rühren geheizt. Bei der anschließenden Vakuumdestillation wurden 138 g (18,9% d. Th., bez. auf Ausgangsgemisch) Toluylendiisocyanat (45% 2,6-, 55% 2,4-Isomeres) erhalten. Der Rückstand (588 g, 80,5 d. Th.) hatte einen NCO-Gehalt von 23,1% und bestand überwiegend aus dem Trimerisat von Toluylendiisocyanat.

### Beispiel 2

1800 g Toluylendiisocyanat (65% 2,4-, 35% 2,6-Isomeres) und 1,75 g Benzoylchlorid wurden unter Stickstoffatmosphäre auf 80°C erhitzt. 5,9 g der Mannichbase gemäß Beispiel 1 der DE-OS 2 551 634 (55%iges Gemisch) wurden zugegeben, die Reaktionstemperatur stieg innerhalb von etw 10 Minuten auf 90°C. Anschließend wurde 1 Stunde bei 90°C gerührt und dann 5 Minuten bei 120°C weiter unter Rühren geheizt. Bei der anschließenden Vakuumdestillation wurden 612 g (34% d. Th., bez. auf Ausgangsgemisch) Toluylendiisocyanat (51,5% 2,6-, 48,5% 2,4-Isomeres) erhalten. Der Rückstand (1188 g, 66% d. Th.) hatte einen NCO-Gehalt von 21,6% und bestand überwiegend aus dem Trimerisat von Toluylendiisocyanat.

### Beispiel 3

Es wurde gearbeitet wie im Beispiel 2 beschrieben, jedoch wurde 2 Stunden bei 90°C gerührt.
Ausbeute (Destillat): 387 g (21,5% d. Th., bez. auf Ausgangsgemisch) Toluylendiisocyanat (59,5% 2,6-, 40,5% 2,4-Isomeres).
Ausbeute (Rückstand): 1413 g (78,5% d. Th.).

5

## Beispiel 4

Es wurde verfahren wie im Beispiel 2 beschrieben, jedoch stieg die Temperatur innerhalb von 30 Minuten auf 90°C und anschließend wurde 10 Min. bei 90°C gerührt.

Ausbeute (Destillat): 135 g (7,5% d. Th., bez. auf Ausgangsgemisch) Toluylendiisocyanat (70% 2,6-, 30% 2,4-Isomeres).

Ausbeute (Rückstand): 1665 g (92,5% d. Th.).

## Beispiel 5

250 g (0,125 Mol) eines Polyetherpolyols aus Propylenoxid und Ethylenoxid (9 : 1, Mol/Mol) mittleren Molekulargewichtes von 2000 wurden 30 Min. bei 120°C i. Vak. entwässert. Bei 120°C wurden 65,3 g (0,375 Mol) Toluylendiisocyanat (70% 2,6-, 30% 2,4-Isomeres) zugegeben, 10 Min. gerührt, dann 3 Tropfen Zinn-II-octoat zugegeben und 20 Min. bei 120°C weitergerührt. Anschließend wurden 21,8 g (0,245 Mol) Butandiol-(1,4) zugegeben, das Gemisch 30 Sek. gerührt und auf eine auf 100°C vorgeheizte Platte (200 × 200 × 60 mm) gegossen.

Nach 24 Stunden Tempern bei 100°C wurde ein Elastomer mit folgenden Eigenschaften erhalten:

| | |
|---|---|
| $\sigma$ 100 | 1,9 MPa (DIN 53504) |
| Zugfestigkeit | 4,5 MPa (DIN 53504) |
| Strukturfestigkeit | 110 N (DIN 53504) |
| Härte | 73 Sh.A (DIN 53505) |
| Elastizität | 43% (DIN 53512) |

## Beispiel 6

250 g (0,125 Mol) eines Polyesterpolyols aus Adipinsäure, Ethylenglykol und Butandiol-1,4 (7 : 3, Mol/Mol) des mittleren Molekulargewichtes 2000 wurden 30 Min. bei 120°C im Vakuum entwässert. Bei 120°C wurden 65,3 g des Diisocyanatgemisches gemäß Beispiel 2 zugegeben und 30 Min. gerührt. Dann wurden 3 Tropfen Zinn-II-octoat und 21,5 g (0,119 Mol) Butandiol-1,4 zugegeben, das Gemisch 30 Sek. gerührt und auf eine auf 100°C vorgeheizte Platte (200 × 200 × 60 mm) gegossen. Nach 24 Stdn. Tempern bei 100°C wurde ein Elastomer mit folgenden Eigenschaften erhalten:

| | |
|---|---|
| $\sigma$ 100 | 0,9 MPa (DIN 53504) |
| Zugfestigkeit | 2,4 MPa (DIN 53504) |
| Strukturfestigkeit | 85 N (DIN 53504) |
| Härte | 62 ShA (DIN 53505) |
| Elastizität | 37% (DIN 53512) |

## Beispiel 7

Es wurde gearbeitet wie im Beispiel 6 beschrieben, jedoch wurde anstelle des dort verwendeten Toluylendiisocyanatgemisches Toluylendiisocyanatgemisch aus 80% 2,6- und 20% 2,4-Isomeren verwendet. Das erhaltene Elastomer besitzt folgende Eigenschaften:

| | |
|---|---|
| $\sigma$ 100 | 2,6 MPa (DIN 53504) |
| Zugfestigkeit | 9,5 MPa (DIN 53504) |
| Strukturfestigkeit | 181 N (DIN 53504) |
| Härte | 74 ShA (DIN 53505) |
| Elastizität | 43% (DIN 53512) |

## Vergleichsbeispiel

Es wurde gearbeitet wie im Beispiel 7 beschrieben, jedoch wurde anstelle des dort verwendeten Toluylendiisocyanatgemisches Toluylendiisocyanatgemisch (»TDI 80«) aus 80% 2,4- und 20% 2,6-Isomeren verwendet. Das erhaltene Elastomer besitzt folgende Eigenschaften:

| | |
|---|---|
| $\sigma$ 100 | 0,3 MPa (DIN 53504) |
| Zugfestigkeit | 0,4 MPa (DIN 53504) |
| Strukturfestigkeit | 33 N (DIN 53504) |
| Härte | 29 Sha (DIN 53505) |
| Elastizität | 18% (DIN 53512) |

## Patentansprüche

1. Verfahren zur Herstellung von im wesentlichen aus 2,4-Diisocyanatotoluol und 2,6-Diisocyanatotoluol bestehenden Diisocyanatgemischen mit einem Gehalt an 2,6-Diisocyanatotoluol, bezogen auf Gesamtgemisch, von 36 bis 90 Gew.-%, dadurch gekennzeichnet, daß man einen Teil der Isocyanatgruppen eines im wesentlichen aus 2,4-Diisocyanatotoluol und 2,6-Diisocyanatotoluol bestehenden Diisocyanatgemischs mit einem Gehalt an 2,6-Diisocyanatotoluol, bezogen auf Gesamtgemisch, von maximal 35 Gew.-% einer Trimerisierungsreaktion unterzieht und aus dem so erhaltenen Reaktionsgemisch ein Diisocyanatgemisch mit einem erhöhten Gehalt an 2,6-Diisocyanatotoluol isoliert.

2. Verfahren zur Herstellung von Polyurethanelastomeren durch Umsetzung von organischen Polyisocyanaten mit höhermolekularen Polyhydroxylverbindungen und Kettenverlängerungsmitteln nach dem Ein- oder Zweistufen-Prinzip, dadurch gekennzeichnet, daß man

a) als Polyisocyanatkomponente ein im wesentlichen aus 2,4-Diisocyanatotoluol und 2,6-Diisocyanatotoluol bestehendes Diisocyanatgemisch mit einem Gehalt an 2,6-Diisocyanatotoluol, bezogen auf Gesamtgemisch, von 36 bis 90 Gew.-% und

b) als Kettenverlängerungsmittel gegebenenfalls durch Etherbrücken unterbrochene aliphatische Glykole des Molekulargewichtsbereichs 62 bis 200,

verwendet.

## Claims

1. A process for the production of diisocyanate mixtures consisting substantially of 2,4-diisocyanato toluene and 2,6-diisocyanato toluene, having from 36 to 90% by weight, based on the total mixture, of 2,6-diisocyanato toluene, characterised in that some of the isocyanate groups of a diisocyanate mixture consisting substantially of 2,4-diisocyanate toluene and 2,6-diisocyanato toluene, having a maximum content of 2,6-diisocyanato toluene of 35% by weight based on the total mixture, is subjected to a trimerisation reaction and from the reaction mixture which is thus obtained, a diisocyanate mixture is isolated having an increased content of 2,6-diisocyanato toluene.

2. A process for the production of polyurethane elastomers by the reaction of organic polyisocyanates with higher molecular polyhydroxyl compounds and chain lengthening agents according to the one stage or two stage principle characterised in that,

a) a diisocyanate mixture consisting substantially of 2,4-diisocyanato toluene and 2,6-diisocyanato toluene, having from 36 to 90% by weight, based on the total mixture, of 2,6-diisocyanato toluene is used as a polyisocyanate component and

b) aliphatic glycols, interrupted optionally by ether bridges, having a molecular weight in the range of from 62 to 200 are used as chain lengthening agents.

## Revendications

1. Procédé de préparation de mélanges de diisocyanates consistant essentiellement en 2,4-diisocyanatotoluène et 2,6-diisocyanatotoluène à une teneur en 2,6-diisocyanatotoluène de 36 à 90% du poids du mélange total, caractérisé en ce que l'on soumet une partie des groupes isocyanate d'un mélange de diisocyanates consistant essentiellement en 2,4-diisocyanatotoluène et 2,6-diisocyanatotoluène à une teneur en 2,6-diisocyanatotoluène de 35% au maximum du poids du mélange total, à une réaction de trimérisation, et à partir du mélange de réaction obtenu on isole un mélange de diisocyanates à teneur accrue en 2,6-diisocyanatotoluène.

2. Procédé de préparation d'élastomères de polyuréthannes par réaction de polyisocyanates organiques avec des composés polyhydroxylés à haut poids moléculaire et des agents d'allongement des chaînes selon le principe en un stade opératoire ou en deux stades opératoires, caractérisé en ce que:

a) on utilise en tant que composant polyisocyanate un mélange de diisocyanates consistant essentiellement en 2,4-diisocyanatotoluène et 2,6-diisocyanatotoluène à une teneur en 2,6-diisocyanatotoluène de 36 à 90% du poids du mélange total, et

b) on utilise en tant qu'agents d'allongement des chaînes des glycols aliphatiques éventuellement interrompus par des ponts éthers, de poids moléculaire 62 à 200.